Europäisches Patentamt

European Patent Office

Office européen des brevets

(11) Veröffentlichungsnummer: **0 308 957**
**A1**

# (12) EUROPÄISCHE PATENTANMELDUNG

(21) Anmeldenummer: **88115673.1**

(22) Anmeldetag: **23.09.88**

(51) Int. Cl.⁴: **A61B 17/22**

(30) Priorität: **24.09.87 DE 3732236**

(43) Veröffentlichungstag der Anmeldung:
**29.03.89 Patentblatt 89/13**

(84) Benannte Vertragsstaaten:
**AT BE CH DE ES FR GB GR IT LI LU NL SE**

(71) Anmelder: **Baumgart, Rainer, Dipl.-Ing. Dr. med.**
**Athener Platz 11**
**D-8000 München 90(DE)**

(72) Erfinder: **Baumgart, Rainer, Dipl.-Ing. Dr. med.**
**Athener Platz 11**
**D-8000 München 90(DE)**

(74) Vertreter: **Selting, Günther et al**
**Patentanwälte Von**
**Kreisler-Schönwald-Fues-Keller**
**Selting-Werner Deichmannhaus am**
**Hauptbahnhof**
**D-5000 Köln 1(DE)**

(54) Rekanalisationskatheter.

(57) Es wird ein Rekanalisationskatheter bereitgestellt, dessen Außenabmessungen die perkutane Anwendung erlauben und mit dem sich Gefäßverschlüsse (2) entfernen lassen, wobei das Lumen (3) des Gefäßes (1)nach dem Fräsen wesentlich größer ist als die Umfangsabmessung des aus einem Zylinder (6) mit einem stirnseitigen Fräselement (7) bestehenden Fräskopfes. In dem Zylinder (6) sind dafür radial auslenkbare Fräsleisten (8) vorgesehen. Das radiale Auslenken erfolgt unter dem Einfluß der Zentrifugalkraft durch Drehzahlsteigerung oder durch axiale Verkürzung des Zylinders (6) bezüglich der Fräsleisten (8), die sich dadurch radial ausbauchen. Nach dem Fräsvorgang werden die Fräsleisten (8) wieder in die Stellung für rein axiales Fräsen zurückgebracht, in der der Rekanalisationskatheter wieder den Durchmesser hat, der das Entfernen durch die perkutane Öffnung erlaubt, die anschließend wegen der geringen Größe ohne Probleme verheilt.

Fig. 1

# Rekanalisationskatheter

Die Erfindung betrifft einen Rekanalisationskatheter nach dem Oberbegriff des Patentanspruchs 1.

Bei einem solchen, aus der EP-A1-0 117 519 bekannten Rekanalisationskatheter bestehen die Fräsleisten aus dünnen, elastischen, Schälmesser bildenden Lamellen, die am Fräskopf und am Führungskatheter festgelegt sind. In der Stellung für ein axiales Fräsen sind die Schälmesser gestreckt und liegen auf einer Zylinderfläche, deren Außendurchmesser im wesentlichen der größten Abmessung des Fräskopfs entspricht. Die biegsame Welle besteht aus einem Spanndraht mit einer um ihn herum angeordneten Wendel. Spanndraht und Wendel sind distal am Fräskopf und proximal an einem Antriebsmotor festgelegt, der in einem Griff angeordnet ist. Mit Hilfe einer Gewindemuffe ist der Abstand zwischen Griff und Führungskatheter veränderbar, wodurch distal der Führungskatheter zum Fräskopf hin verschiebbar oder von diesem wegbewegbar ist, wodurch die Schälmesser aus hochelastischem Material in die Schälstellung aufgewölbt oder in die Stellung in der Zylinderfläche flachgestreckt werden können. Die um den Spanndraht herum angeordnete Wendel dient dazu, daß von den Schälmessern abgeschälte Material durch den Führungskatheter hindurch zu dessen proximalen Ende zu transportieren.

Der bekannte Rekanalisationskatheter kann durch das Ausfahren von Schälmessern zwar Lumina fräsen, die größer als der axiale Fräskopf sind, das Abtragungsprinzip ist jedoch nicht geeignet für die Anwendung in arteriosklerotisch verschlossenen Gefäßen, da der bekannte Rekanalisationskatheter in gleicher Weise Verschlußmaterial und Gefäßwand abfräst und so ein erhebliches Perforationsrisiko besteht. Der bekannte Rekanalisationskatheter ist darüber hinaus störanfällig, da die Schälmesser sehr dünn sind, sich leicht verwinden können und die Gefahr einer Verstopfung des Abführkanals mit abgeschältem Material besteht, was die Rückführung der Schälmesser in ihre flachgestreckte Führungsstellung in der Zylinderfläche beeinträchtigt und somit das Entfernen des Rekanalisationskatheters durch eine perkutane Schleuse verhindert.

Ferner besteht die Gefahr, daß bei einer Anordnung, bei der die Schälmesser mit ihrer Schmalseite auf das kalkharte, wandadhärente Verschlußmaterial treffen, durch Verhaken die Gefäßwand mitgerissen wird und somit folgenschwere Schäden auftreten.

Die der Erfindung zugrunde liegende Aufgabe besteht deshalb darin, den Rekanalisationskatheter der gattungsgemäßen Art für die perkutane Anwendung so auszubilden, daß die Fräsleisten möglichst selektiv das Verschlußmaterial auf einen Durchmesser entfernen, der nahezu dem ursprünglichen Gefäßlumen entspricht, ohne in gleicher Weise die Gefäßwand anzugreifen, wobei das radiale Ausfahren der Fräsleisten aus der Zylinderfläche und ihre Rückführung in die oder untor die Zylinderfläche störungsfrei gewährleistet ist und die Gefäßwand nicht mitgerissen wird.

Diese Aufgabe wird mit den im kennzeichnenden Teil des Patentanspruchs 1 angegebenen Merkmalen gelöst.

Der erfindungsgemäße Rekanalisationskatheter kann mit einem Durchmesser von nicht mehr als 2,5 mm gebaut werden, so daß seine perkutane Einführung in das Gefäß durch eine Schleuse erfolgen kann, nach deren Entfernung, wenn der Gefäßverschluß beseitigt worden ist, sich die Gefäßöffnung von selbst schließt, ohne daß operative Maßnahmen erforderlich sind. Für ein axiales Fräsen betragen die Drehzahlen mindestens 5000 Upm. Für das radiale Fräsen können die Drehzahlen auf über 50000 Upm gesteigert werden. Mit den Fräsleisten des erfindungsgemäß ausgestalteten Rekanalisationskatheters erfolgt kein Abschälen des Materials des Gefäßverschlusses, sondern eine Zerkleinerung des Verschlußmaterials auf Teilchengrößen, bei denen unter Mitwirkung der verwendeten thrombolytisch wirkenden Kühl- und Spülflüssigkeit nur eine geringe Emboliegefahr besteht. Obwohl sich mit fortschreitenden pathologischen Veränderungen die mechanischen Eigenschaften von Verschlußmaterial (eher isotrop, amorph) und Gefäßwand (eher anisotrop, elastisch) einander annähern, bleibt ein Restunterschied, weshalb eine stumpfe, sich schnell drehende Fräsleiste das amorphe Material abtragen kann, während sie von dem restelastischen Wandmaterial abprallt und somit zentriert wird.

Aufgrund des Einsatzes der Spülflüssigkeit ist eine Verstopfung des Raums in den Längsnuten nicht möglich, so daß die zugehörige stabil ausgebildeten, sich beim Fräsen nicht verformenden oder verbiegenden Fräsleisten sicher entweder durch den Druck der Gefäßwand, durch die elastischen Rückstellkräfte im Biegegelenk oder durch Rückstellelemente innerhalb der Zylinderfläche des Rekanalisationskatheters liegend zurückgeführt werden können, wodurch er problemlos durch die Schleuse entfernt werden kann.

Die Festlegung der Fräsleisten am Zylinder gemäß Anspruch 2 ist funktionsgerecht und konstruktionsmäßig äußerst einfach.

In der Ausgestaltung des Rekanalisationskatheters nach Anspruch 3 wird mit einfachen Mitteln abhängig vom Fluiddruck das Ausfahren der Fräsleisten in die Stellung für radiales Fräsen gewährleistet.

Dieses Ausfahren erfolgt mit der Ausgestaltung nach Anspruch 4 durch den Einfluß der Zentrifugalkraft, wofür lediglich eine entsprechende Drehzahleinstellung vorgenommen zu werden braucht.

Durch die Ausgestaltung nach Anspruch 5 wird ein maximaler radialer Fräsradius festgelegt.

Die in Anspruch 6 angegebene Maßnahme ermöglicht eine sichere Rückführung der Fräsleisten in die Stellung innerhalb der Zylinderfläche für rein axiales Fräsen oder für das Entfernen des Rekanalisationskatheters aus dem Gefäß, wenn die Rückstellkräfte des elastischen Biegegelenks nicht genügen sollten und die Gefäßwand keine Rückstellkraft ausüben soll. Als Rückstellelemente eignen sich beispielsweise Schraubenfedern.

In der Ausgestaltung der Ansprüche 7 und 8 lassen sich die Fräsleisten biegegelenkseitig in einfacher Weise am Zylinder festlegen, wobei an diesem zur Aufrechterhaltung eines gleichbleibenden Durchmessers lediglich eine entsprechende Verringerung seines Außendurchmessers durch Abdrehen vorgenommen zu werden braucht.

Die Ausgestaltung nach Anspruch 9 gewährleistet, daß beim radialen Fräsen das Spülfluid hauptsächlich radialseitig austritt, während beim axialen Fräsen das Spülfluid lediglich stirnseitig austritt, ohne daß zusätzlich konstruktive Mittel für den Verschluß der radialen Austrittsöffnungen vorgesehen werden müssen.

Anhand von Zeichnungen wird ein Ausführungsbeispiel der Erfindung näher erläutert. Es zeigt:

Fig. 1 perspektivisch einen Rekanalisationskatheter innerhalb eines teilweise aufgeschnittenen, innen verschlossenen Gefäßes,

Fig. 2 den Rekanalisationskatheter von Fig. 1 im Axialschnitt in der Stellung für rein axiales Fräsen,

Fig. 3 eine Stirnansicht des Rekanalisationskatheters von Fig. 2,

Fig. 4 in einer Ansicht wie Fig. 2 den Rekanalisationskatheter in der Stellung für radiales Fräsen und

Fig. 5 den Rekanalisationskatheter von Fig. 4 in der Stirnansicht.

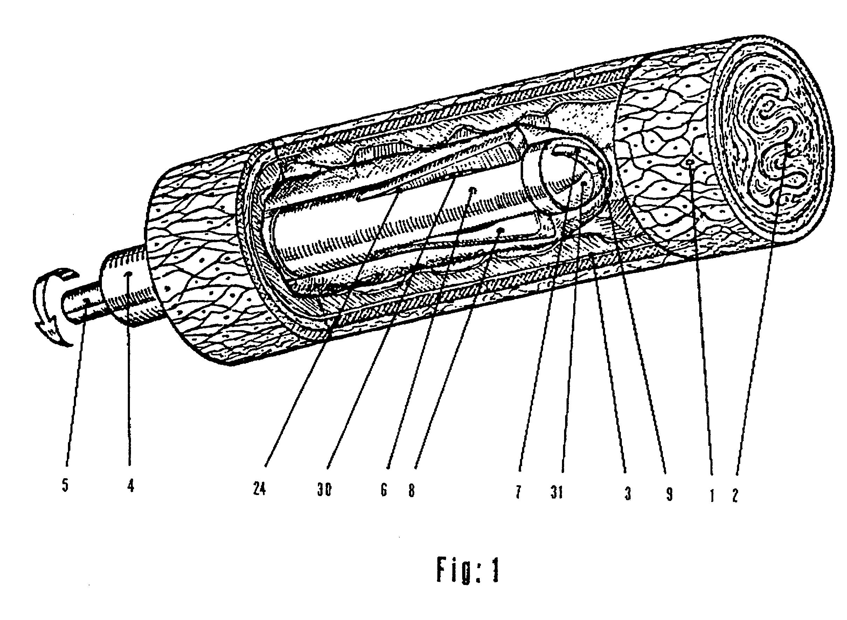

Der in den Fig. 1 bis 5 gezeigte Rekanalisationskatheter hat eine biegsame, drehangetriebene Welle 5, die sich durch einen rohrförmigen flexiblen Führungskatheter 4 hindurch zu einem Zylinder 6 erstreckt, an dem stirnseitig ein halbkugelförmiger Fräskopf 31 mit einem Fräselement 7 für axiales Fräsen angeordnet ist. Das distale Ende der biegsamen Welle 5 ist drehfest mit einer sie umschließenden Wellenhülse 25 verbunden. Zwischen der Welle 5 sowie der Wellenhülse 25 und dem Führungskatheter 4 ist ein ringförmiger Fluidkanal 10 vorhanden, der über eine Umfangsnut 22 in der Wellenhülse 25 und eine Bohrung in ihrer dem Zylinder 6 zugewandten Stirnseite mit einem axialen Fluidkanal 23 im Zylinder 6 verbunden ist. Von der dem Zylinder 6 zugewandten Stirnseite der Wellenhülse 25 erstreckt sich ein koaxialer Hülsenansatz 17, der mit einem zylindrischen Fortsatz 16 des Zylinders 6, durch den sich auch der axiale Fluidkanal 23 erstreckt, drehfest verbunden ist. In das distale Ende des Führungskatheters 4 ist eine Lagerhülse 18 eingesetzt, die mit einer Schulter 19 an der Ringfläche des Führungskatheters 4 anliegt und deren Umfangsfläche mit der Umfangsfläche des Führungskatheters 4 fluchtet. Die Innenfläche der Lagerhülse 18 und die Außenfläche des Hülsenansatzes 17 bilden den Lagerspalt 20 zwischen dem nichtrotierenden Führungskatheter 4 und dem Zylinder 6, wenn dieser über die biegsame Welle 5 in Drehung versetzt wird. Für die Schmierung und Kühlung der Lagerung sorgt in den Lagerspalt 20 eindringende Spülflüssigkeit, die beim Austritt aus dem Ringspalt zwischen der Schulter 19 und dem Zylinder 6 den Rekanalisationskatheter zusätzlich zentriert. Der axiale Fluidkanal 23 endet in einer zentralen Fluidaustrittsöffnung 9 im Fräskopf 31, an deren Stelle auch mehrere, am Umfang verteilte Fluidauslässe vorgesehen werden können.

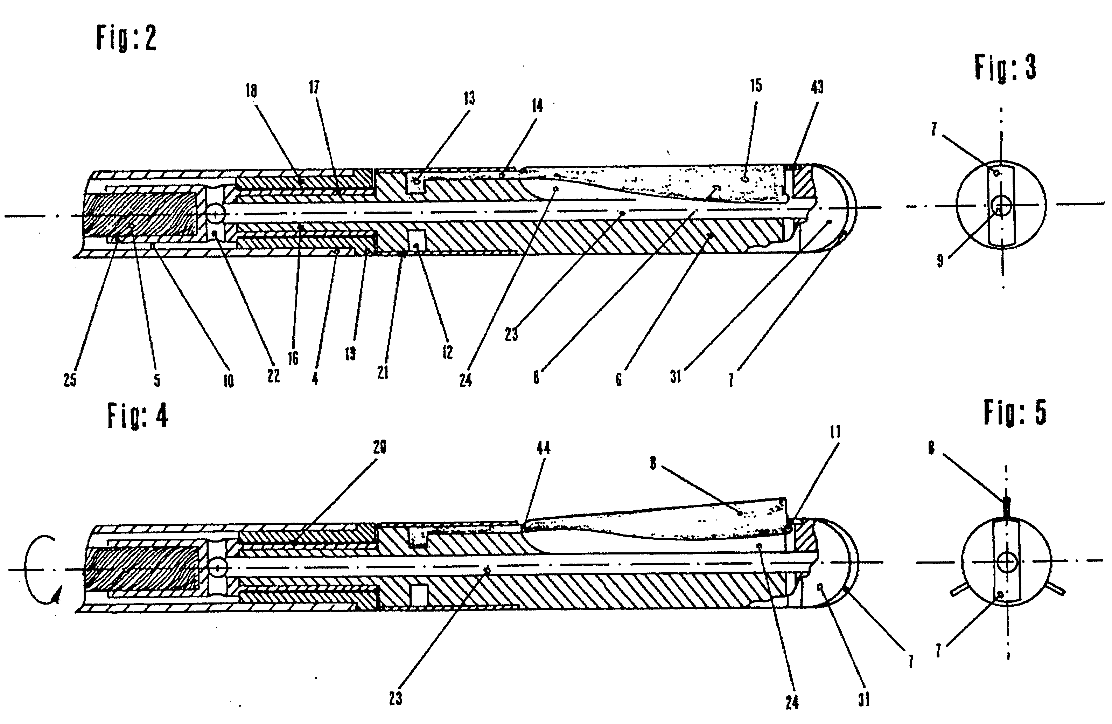

Wie aus Fig. 1 zu ersehen ist, dient der Rekanalisationskatheter dazu, einen in einem Blutgefäß 1 vorhandenen Verschluß 2 durch Fräsen zu beseitigen, um so nahezu das ursprüngliche Lumen 3 des Gefäßes wieder zu erhalten. Im Zylinder 6 sind dafür in einem Winkelabstand von 120° jeweils eine Längsnut 24 vorgesehen, an die sich zum Führungskatheter 4 hin eine Vertiefung in Form einer Ringnut anschließt. In der Vertiefung sitzt jeweils ein Fuß 13 einer Fräsleiste 8, die über einen in der Längsnut 24 liegenden Steg 14 und über ein Biegegelenk 44 in einen radialen Fräsabschnitt 15 übergeht, der ebenfalls in der Längsnut 24 sitzt und dessen Masse zum Fräselement 7 hin zunehmend ausgebildet ist. Durch eine Haltebüchse 21 werden der Fuß 13 in der Vertiefung und der Steg 14 in seiner Längsausnehmung im Zylinder 6 axial fixiert gehalten, wobei die Außenumfangs fläche der Haltebüchse 21 mit der des Führungskatheters 4 fluchtet. Bei der in Fig. 2 und 3 gezeigten Stellung für rein axiales Fräsen befindet sich der Fräsabschnitt 15 innerhalb der Längsnut 24, so daß seine Außenfläche innerhalb der Zylinderfläche liegt.

Bei einer Steigerung der Wellendrehzahl über eine bestimmte Drehzahl hinaus wird jeder Fräsabschnitt 15 um sein Biegegelenk 44 mit seinem massereicheren fräskopfseitigen Ende aufgrund der Zentrifugalkraft radial nach außen aus der Längsnut 24 heraus über die Zylinderfläche verschwenkt, bis eine Nase 11 am Fräsabschnitt 15 an einem Anschlag 43 am Zylinder 6 im Bereich der Zylinderflächen zum Anliegen kommt, wodurch die radiale Auslenkung des Fräsabschnitts 15 und somit der maximale Fräsdurchmesser begrenzt sind. Die Kontaktflächen zwischen den Fräsabschnitten 15 und den Längsnuten 24 werden beim Auslenken der Fräsleisten 8 kontinuierlich kleiner, so daß vermehrt Spülflüssigkeit durch den Spalt in Radialrichtung austreten kann. Nach dem Abschalten des Drehantriebs am proximalen Ende der biegsamen Welle 5 kehren die Fräsleisten 8 aus der in Fig. 4 gezeigten radialen Frässtellung in die in Fig. 2 gezeigte Stellung für rein axiales Fräsen innerhalb der Mantelfläche des Zylinders 6 zurück, wofür zusätzlich nicht gezeigte elastische Rückstellelemente vorgesehen werden können, falls die elastische Rückstellkraft am Biegegelenk 44 nicht genügt. Der Durchmesser des Rekanalisationskatheters ist dadurch wieder auf den Durchmesser für rein axiales Fräsen zurückgeführt, so daß er problemlos durch die Einführungsschleuse herausgezogen werden kann, nach deren Entfernung sich wegen des geringen Öffnungsdurchmessers die Gefäßpunktionsstelle nach kurzer Kompression verschließt.

## Ansprüche

Rekanalisationskatheter mit einer biegsamen, in einem nicht drehenden rohrförmigen flexiblen Führungskatheter (4) angeordneten, drehangetriebenen Welle (5), an der ein Fräskopf (31) mit wenigstens einem stirnseitig angebrachten Fräselement (7) befestigt ist, mit zwischen dem Fräskopf (31) und dem Führungskatheter (4) vorgesehenen, mit dem Fräskopf (31) in Drehung versetzbaren, massegleichen, in gleichen Winkelabständen angeordneten Fräsleisten (8), die in einer Stellung für rein axiales Fräsen im wesentlichen innerhalb oder in einer Zylinderfläche liegen und in eine Stellung für radiales Fräsen aus der Zylinderfläche herausführbar sind, und mit einem im Führungskatheter (4) vorgesehenen Kanal (10), dadurch **gekennzeichnet**, daß die Welle (5) mit der einen Stirnseite eines Zylinders (6) verbunden ist, an dessen anderer Stirnseite der Fräskopf (31) sitzt, in dem ein mit dem Kanal (10) im Führungskatheter (4) verbundener, Austrittsöffnungen (9, 30, 24) aufweisender zentraler Fluidkanal (23) ausgebildet ist, und in dessen Zylinderfläche Längsnuten (24) ausgespart sind, daß die Fräsleisten (8) an ihrem führungskatheterseitigen Ende am Zylinder (6) festgelegt sind, mit ihrem sich zum Fräskopf (31) hin erstreckenden Fräsabschnitt (15) in der Stellung für rein axiales Fräsen innerhalb der Längsnut (24) liegend angeordnet sind und für das radiale Fräsen aus den Längsnuten (24) über die Zylinderfläche hinaus auslenkbar sind.

2. Rekanalisationskatheter nach Anspruch 1, dadurch **gekennzeichnet**, daß die Fräsleisten (8) an ihrem führungskatheterseitigen Ende über ein Biegegelenk (44) am Zylinder (6) festgelegt sind.

3. Rekanalisationskatheter nach Anspruch 1 oder 2, **gezeichnet** durch einen zentral im Zylinder (6) innerhalb der Fräsabschnitte (15) der Fräsleisten (8) in deren Stellung für rein axiales Fräsen angeordneten, an den Führungskanal (23) angeschlossenen, durch das Fluid aufblähbaren Ballon.

4. Rekanalisationskatheter nach Anspruch 1 oder 2, dadurch **gekennzeichnet**, daß der Fräsabschnitt (15) jeder Fräsleiste (8) eine zum Fräskopf (31) hin zunehmende Masse aufweist.

5. Rekanalisationskatheter nach einem der vorhergehenden Ansprüche , dadurch **gekennzeichnet**, daß der Fräsabschnitt (15) jeder Fräsleiste (8) fräskopfseiti eine Nase (11) aufweist, der ein die radiale Auslenkung des Fräsabschnitts (15) begrenzender zylinderfester Anschlag (43) zugeordnet ist.

6. Rekanalisationskatheter nach einem der Ansprüche 1 bis 5, **gekennzeichnet** durch dem Fräsabschnitt (15) jeder Fräsleiste (8) zugeordnete, federnd elastisch wirkende, für die Aufhebung ihrer radialen Auslenkung vorgespannte Rückstellelemente.

7. Rekanalisationskatheter nach einem der Ansprüche 2 bis 6, dadurch **gekennzeichnet**, daß die Biegegelenke (44) der Fräsleisten (8) führungskatheterseitig durch einen außenseitig die Zylinderfläche bildenden Ringsteg am Zylinder (6) festgelegt sind.

8. Rekanalisationskatheter nach einem der Ansprüche 2 bis 6, dadurch **gekennzeichnet**, daß jede Fräsleiste (8) zum Führungskatheter hin anschließend an das Biegegelenk (44) einen Steg (14) und einen Fuß (13) aufweist und daß die Füße (13) der Fräsleisten (8) in eine Ringnut im Zylinder (6) eingreifen und in dieser Eingriffsstellung durch eine über sie und die Stege (14) geschobene, außenseitig die Zylinderfläche bildende Haltebüchse (21) gehalten sind.

9. Rekanalisationskatheter nach einem der vorhergehenden Ansprüche, dadurch **gekennzeichnet**, daß die Fräsleisten (8) in ihrer Stellung für rein axiales Fräsen Verschlüsse für die radial vorgesehenen Austrittsöffnungen (30, 24) für das Fluid bilden.

Fig: 1

5    4        24    30    6  8        7  31      3   9   1  2

EP 0 308 957 A1

Fig: 2

Fig: 3

Fig: 4

Fig: 5

EP 0 308 957 A1

Europäisches
Patentamt

**EUROPÄISCHER RECHERCHENBERICHT**

Nummer der Anmeldung

EP 88 11 5673

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 117 519 (THEERMANN)<br>* Insgesamt *<br>--- | 1 | A 61 B 17/22 |
| A | US-A-2 730 101 (HOFFMAN)<br>* Figuren; Anspruch 1 *<br>--- | 1 | |
| A | GB-A-2 157 207 (MIYANAGA)<br>--- | | |
| A | US-A-2 788 621 (DOYLE)<br>------ | | |

**RECHERCHIERTE SACHGEBIETE (Int. Cl.4)**

A 61 B
B 23 B
B 24 B

Der vorliegende Recherchenbericht wurde für alle Patentansprüche erstellt

| Recherchenort | Abschlußdatum der Recherche | Prüfer |
|---|---|---|
| DEN HAAG | 16-01-1989 | STEENBAKKER J. |

**KATEGORIE DER GENANNTEN DOKUMENTE**

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
    ...........................................................................
& : Mitglied der gleichen Patentfamilie, übereinstimmendes
    Dokument

EPO FORM 1503 03.82 (P0403)